Europäisches Patentamt

(19) European Patent Office

Office européen des brevets

(11) Veröffentlichungsnummer: **0 015 876**
**A1**

(12) **EUROPÄISCHE PATENTANMELDUNG**

(21) Anmeldenummer: **80810072.1**

(22) Anmeldetag: **27.02.80**

(51) Int. Cl.³: **C 07 C 39/08, C 07 C 37/05**

(30) Priorität: **01.03.79 CH 2045/79**

(43) Veröffentlichungstag der Anmeldung: **17.09.80**
**Patentblatt 80/19**

(84) Benannte Vertragsstaaten: **AT BE DE FR GB IT NL SE**

(71) Anmelder: **ELPROCHINE AG, Hochstrasse 28, CH-8044 Zürich (CH)**

(72) Erfinder: **Biller, Efim, Hochstrasse 28, CH-8044 Zürich (CH)**

(54) **Verfahren zur Herstellung von Hydrochinon.**

(57) Hydrochinon wird durch saure Hydrolyse von p-Aminophenol in wässeriger Lösung von organischen Sulfonsäuren bzw. der p-Aminophenol Salze organischer Sulfonsäure der sauren katalytischen oder elektrolytischen Reduktion von Nitrobenzol bei Temperaturen von 190—300 °C und 0,01—10 Stunden Reaktionszeit hergestellt, durch Extraktion mit hydrophoben Lösungsmitteln gewonnen und die wässerigen Sulfonsäure-Lösungen nach mindestens teilweiser Entfernung des Ammoniaks durch Ionen-Austauscher oder über wasserunlösliche Schwefelsäure-Salze im Kreis geführt.

EP 0 015 876 A1

ELPROCHINE AG, Zürich

---

Verfahren zur Herstellung von Hydrochinon

------------------------------------------------

Die Erfindung betrifft ein Verfahren zur Herstellung von Hydrochinon durch saure Hydrolyse von p-Aminophenol in wässeriger Lösung von organischen Sulfonsäuren bzw. der p-Aminophenol-Salze organische Sulfonsäuren der sauren katalytischen oder elektrolytischen Reduktion von Nitrobenzol.

Hydrochinon wird großtechnisch durch Oxydation von Anilin in Schwefelsäure mit Braunstein bzw. Natriumbichromat zur Chinon und anschließenden Reduktion mit Eisen bzw. Schwefeldioxid in Wasser hergestellt.

Die DOS 2.459233 beschreibt ein Verfahren zur Hydrolyse von p-Aminophenol mit wässeriger Lösung eines Ammoniumbisulfates, wobei das bei der Hydrolyse gebildete Ammoniumsulfat nach Abdampfen des Wassers thermisch in Ammoniumbisulfat umgewandelt und nach Verdünnung mit Wasser und Filtration der Harze wieder verwendet wird.

Das obigen Verfahren weist verschiedene Nachteile auf:

1) Während der Hydrolyse bilden sich Feststoffe (Harze)

2) Die Ausbeute an Hydrochinon liegt bei ca. 70 %

3) Es muß mit hohen Wassermengen gearbeitet werden, da es sonst zur Ausscheidung von Ammoniumbisulfat kommt.

4) Zur Erreichung von gutem Umsetzen müssen 2-3 Mol Ammoniumbisulfat pro 1 Mol p-Aminophenol eingesetzt werden.

5) Beim Arbeiten mit Ammoniumbisulfat müssen ca. 15-17 Tonnen Wasser pro/t Hydrochinon verdampft werden.

Die DOS 2.601146 beschreibt Hydrolyse von p-Aminophenol zur Hydrochinon mit Schwefelsäure und Ortho-Phosphorsäure.

Auch diese Verfahren haben Nachteile:

1) Schwefelsäure führt zur Bildung von Nebenprodukten;

2) Phosphorsäure muß in hohen Mengen eingesetzt werden und ergibt schlechte Ausbeuten.

Aufgabe der vorliegenden Erfindung war es daher, ein Verfahren zur Herstellung von Hydrochinon durch saure Hydrolyse von p-Aminophenol zur Verfügung zu stellen, das nicht mehr die genannten Nachteile aufweist.

Diese Aufgabe wurde dadurch gelöst, daß man die Hydrolyse von p-Aminophenol mit wässerigen organischen Sulfonsäuren durchführt und die dabei gebildete Ammoniumslaze entweder durch Ionenaustausch oder über Calcium bzw. andere Wasser-unlösliche Salze in freie Säure umwandelt und im Kreis führt.

Gegenstand der vorliegenden Erfindung ist somit ein Verfahren zur Herstellung von Hydrochinon durch saure Hydrolyse von p-Aminophenol, dadurch gekennzeichnet, daß man:

a) p-Aminophenol und/oder p-Aminophenol-Salze, Sauren Katalytischen oder Elektrolytischen Reduktion von Nitrobenzol in 0,5 - 30 vorzugsweise 3 - 15 % wässeriger Lösung mit 0,3 - 3 vorzugsweise 0,6 - 1,5 Mol organischer Sulfonsäuren pro Mol p-Aminophenol bei Temperaturen von 190 - 300 vorzugsweise 200 - 265 °C, Verweilzeiten von 0,01 - 10 h, vorzugsweise 0,1 - 3 h pro Durchgang, in einen oder mehreren Durchgängen, mit Entfernung des gebildeten Hydrochinons nach jedem Durchgang mit hydrophoben Lösungsmitteln, hydrolysiert und die Extrakte in bekannter Weise aufarbeitet.

b) Die hydrochinonfreie wässerige Lösung direkt oder nach pH-Einstellung auf ca. 5 gegebenenfalls nach Behandlung mit Filtriermitteln, mit Ionenaustauschern oder über Schwefelsäure unlösliche Salze von Ammoniak ganz oder teilweise befreit und die wässerige Säure-Lösung im Kreis führt.

Es hat sich gezeigt, daß organische Sulfonsäuren bei Hydrolyse von p-Aminophenol praktisch keine Nebenreaktionen zeigen, was darauf zurückzuführen ist, daß diese praktisch indifferent sind.

Als Säure können alle organischen Sulfonsäuren angewendet werden die folgende Kriterien aufweisen:

1) Die Stabilität unter Hydrolysebedingungen. Es ist bekannt, daß bestimmte Sulfonsäuren bei höheren Temperaturen (über 150 °C) zur Desulfonierung neigen. (Ullmanns Encyklopädie der Technischen Chemie, 3.Auflage, Band 16, Seite 543).

2) Genügende Löslichkeit des p-Aminophenol Salzes in Wasser. (E.Chambers, G.W.Watt, J.Org.Chem.6,376 (1941).

Anwendbar sind: Alkyl, Benzol, Boluol, Toluol und ähnliche Mono- und Disulfonsäuren, insbesondere Methan und Äthansulfonsäuren sowie Trifluormethansulfonsäure.

Die Reaktionszeit ist von der Temperatur abhängig: mit Erhöhung der Temperatur verkürzt sich die Reaktionszeit zum Erreichen des Gleichgewichtes.

Die Konzentration des p-Aminophenols kann z.B. bei Anwendung von Methansulfonsäure im Bereich von 0,5 - 30 % vorzugsweise 3 - 15 % in der Lösung sein.

Die Reaktionstemperatur kann im Bereich von 190 - 300 °C variieren, da unter 200 °C die Reaktion zu langsam, über 300 °C wegen Erhalten von flüssiger Phase zu hohen Wasserdampfdruck führt (über 105 atm). Bevorzugt wird der Temperaturbereich von 200 - 265 °C.

In Abhängigkeit von Temperaturen liegen die Hydrolysezeiten im Bereich von 0,01 - 10 h, vorzugsweise 0,1 - 3 h.

Als hydrophobe Lösungsmittel kommen praktisch alle Wasser unlöslichen Lösungsmittel, die für Hydrochinon genügende Löslichkeit aufweisen, in Frage, insbesondere Äther, Alkohole und Ester.

Auch bei höheren Temperaturen kann das Hydrochinon extrahiert werden. Hier können auch Aromaten wie z.B. Benzol und Alkylbenzole sowie z.B. Diphenyläther verwendet werden.

Die Hydrolyse kann in einem oder mehreren Durchgängen erfolgen um das p-Aminophenol maximal zu Hydrochinons umzusetzen.

Das p-Aminophenol kann nach beliebigen Prozessen hergestellt werden.

Von besonderer Bedeutung ist die Möglichkeit das p-Aminophenolsalz der organischen Sulfonsäuren direkt aus Nitrobenzol durch katalytische oder elektrolytische Reduktion in wässerigen Lösungen der organischen Sulfonsäuren herzustellen und diese nach Entfernen von nichtumgesetzten Nitrobenzol bzw. Katalysators und Einstellung auf die notwendige Konzentrationen direkt zur Hydrolyse einzusetzen.

Die bei der Reduktion sich bildenden Nebenprodukte wie Anilin und Diaminodiphenyläther stören die Hydrolyse nicht: Anilin bleibt unverändert und das Diaminodiphenyläther wird zu p-Aminophenol hydrolysiert.

Bei der Aufarbeitung der Hydrolysate kann das Anilin durch Einstellung des pH auf ca. 5 freigemacht und gemeinsam mit Hydrochinon oder extra nach dem Stand der Technik gewonnen werden.

Bei der Hydrolyse mit Sulfonsäuren bilden sich in Unterschied zum Arbeiten nach bekannten Verfahren keine unlöslichen Stoffe, ausgenommen Spuren von Oxydationsprodukten und Polymeren die zur Verfärbung der Hydrolyselösung führen.

Es wurde überraschenderweise gefunden, daß diese Verfärbungen mit Äther bzw. Ketone und Ester nicht zu entfernen sind.

Dagegen ist es möglich durch Extraktion mit wasserunlöslichen Alkoholen $C_4$ und höher, die gelösten Verunreinigungen zu entfernen, wodurch man eine reine wässerige Lösung von Ammoniumsulfonsäuresalzen sowie nichtumgesetzten p-Aminophenolsulfonat erhält.

Von Vorteil ist es, die Extraktion des Hydrochinon mit $C_4$ und höheren Alkoholen durchzuführen, wobei gleichzeitig die Entfärbung erfolgt.

Die Entfernung von Verfärbungen ist von Bedeutung, insbesondere wenn man die Rückführung der Säure über Ionenaustausch führt.

Das nicht-umgesetzte p-Aminophenol kann auch auf konventionelle Weise rückgewonnen werden, indem man die Hydrolyselösung vor oder nach der Hydrochinon-Extraktion mit Base ($NH_3$, NaOH oder andere) auf pH 7 - 7,2 einstellt. Das p-Aminophenol liegt dann in freier Form und kann aus der Lösung entfernt werden.

Die Tatsache, daß die Ca und Ba-Salze von organischen Sulfonsäuren wasserlöslich sind, wird bereits in der Technik benützt um die Trennung dieser Säure von Schwefelsäure zu erreichen.

Durch Umwandlung des Ammoniumsulfonates in z.B. Calciumsulfonat wird $NH_3$ frei und kann entfernt werden.

Anschließend wird das Ca-Sulfonat mit Schwefelsäure zum Ca-Sulfat umgewandelt und durch Filtration entfernt. Man erhält wässerige Sulfonsäure, die in die Hydrolyse zurückgeführt wird.

Das Verfahren kann kontinuierlich und/oder diskontinuierlich betrieben werden.

Bei Aufarbeitung der Säure-Lösungen mit Ionenaustauscher ist es wichtig, daß die Lösungen sehr rein sind. Es kann von Vorteil sein, vor dem Ionenaustausch die wässerigen Lösungen über Filtriermittel wie Aktivkohle, Bleicherden und ähnliche, die nach Möglichkeit Sauerstoff-frei sind, vorzubehandeln.

Beim Arbeiten mit Ionenaustauschern bleibt das p-Aminophenolsulfonat als Salz unverändert, dagegen wird das Ammoniak größtenteils gebunden.

Dieses ist auf die höhere Basizität des Ammoniaks zurückzuführen.

## Beispiele

### Beispiel 1

109 gr (1 Mol) p-Aminophenol, 96 gr Methansulfonsäure (1 Mol) und 885 gr Wasser (10 % p-Aminophenollösung) wurden in eine 1,5 l fassende Glasbombe gegeben und in einem Autoclaven mit Wasser gefüllt, auf 240 °C erhitzt und 3 h reagieren lassen.

Die schwarzgefärbte, aber keine Feststoffe enthaltene Lösung wird abgekühlt und mit Äther extrahiert.

Es wurden 72,0 gr Rohhydrochinon erhalten, das nach Redestillation in Vakuum 71,5 gr Gaschromatographisch (GC) Reine Produkt ergibt.

Die wässerige Lösung wird von gelöstem Äther befreit und wieder 3 h bei 240 °C hydrolysiert. Die Extraktion liefert 25,0 gr Rohprodukt, das durch Redestillation 24,5 gr.GC Reines Hydrochinon ergibt. Durch Analyse wurde festgestellt, daß in der wässerigen Lösung noch 12,5 gr p-Aminophenol enthalten sind.

Die Bilanz sieht folgendermaßen aus:

|  | 1 Hydrolyse | 2 Hydrolyse |
|---|---|---|
| Umsatz | 65,13 % | 68,0 % |
| Ausbeute, Reines Hydrochinon | 99,3 % | 99,0 % |

Gesamtbilanz:

| Umsatz | 88,53 % |
|---|---|
| Ausbeute | 99,0 % |

Die extrahierte, schwarzgefärbte, wässerige Lösung wird mit n-Amylalkohol unter Stickstoff extrahiert.

Die wässerige Lösung ist praktisch wasserklar und kann durch Ionenaustauscher in freie Säure umgewandet werden.

### Beispiel 2

Zum Vergleich mit dem Beispiel der DOS 2.459 233 Seite 7 wurde folgender Versuch gemacht:

| Einsatz: | DOS 2 459 233 | Erfindergemäß |
|---|---|---|
| p-Aminophenol | 32,7 gr (0,3 Mol) | 32,7 gr (0,3 Mol) |
| $NH_4 HSO_4$ | 69,0 gr (0,6 Mol) | - |
| $CH_3SO_3H$ | - | 28,8 gr (0,3 Mol) |
| $H_2O$ | 420 gr | 460 gr |
| Konzentrat.p-Aminophenol | 6,27 % | 6,27 % |

Beide Ansätze wurden in Glasbomben 3 h bei 240 gehalten.

Erfindungsgemäßes Beispiel wurde gleich wie das Vergleichsbeispiel aufgearbeitet.

Es wurden folgende Resultate erhalten:

|  | DOS 2 459 233 | | Erfindergemäß | |
|---|---|---|---|---|
| Feststoffe | 1,4 gr | 4,25 % | – | |
| Rohprodukt | 27,0 gr | 81,8 % | 28,2 gr | 85 % |
| Hydrochinon Rein | 24,0 gr | 72,7 % | 28,0 gr | 84,8 % |
| Rückstand | 2,0 | 6,1 % | 0,2 | 0,2 % |

Wasserverdampfung
auf 1 t Hydrochinon     17.320 kg          :      ———

PATENTANSPRÜCHE

1) Verfahren zur Herstellung von Hydrochinon aus para Aminophenol oder Nitrobenzol, dadurch gekennzeichnet, daß man:

a) p-Aminophenol und/oder p-Aminophenol-Salze der sauren katalytischen oder Elektrolytischen Reduktion von Nitrobenzol, in 0,5 - 20 vorzugsweise 3 - 15 % wässeriger Lösung mit 0,3 - 3 vorzugsweise 0,5 - 1,5 Mol organischer Sulfonsäure pro Mol p-Aminophenol bei Temperaturen von 190 - 300 vorzugsweise 200 - 265 °C, Verweilzeiten von 0,01 - 10 h, vorzugsweise 0,1 - 3 h pro Durchgang, in einen oder mehreren Durchgängen, mit Entfernung des gebildeten Hydrochinons nach jedem Durchgang mit Hydrophoben Lösungsmitteln, hydrolysiert und die Extrakte in bekannter Weise aufarbeitet.

b) Die hydrochinonfreie wässerige Lösung direkt oder nach pH-Einstellung auf ca. 5 gegebenenfalls nach Behandlung mit Filtriermitteln, mit Ionenaustauschern oder über Schwefelsäure unlösliche Salze, von Ammoniak ganz oder teilweise befreit und die wässerige Säure-Lösung im Kreis führt.

2) Verfahren nach Anspruch 1, dadurch gekennzeichnet, das beim Arbeiten mit p-Aminophenolsalz-Lösungen der katalytischen oder elektrolytischen Reduktion das nichtumgesetzte Nitrobenzol vor der Hydrolyse entfernt.

3) Verfahren nach den Ansprüchen 1 bis 2, dadurch gekennzeichnet, das als Hydrophobe Lösungsmittel Alkohole mit $C_4$ und höher verwendet

4) Verfahren nach Anspruch 1 bis 3, dadurch gekennzeichnet, daß man mit Alkylsulfonsäuren $C_{1-4}$ arbeitet.

5) Verfahren nach Anspruch 1 bis 4, dadurch gekennzeichnet, daß man mit Trifluorsulfonsäure arbeitet.

| | Europäisches Patentamt | EUROPÄISCHER RECHERCHENBERICHT | | Nummer der Anmeldung<br>EP 80 81 0072 |
|---|---|---|---|---|

| EINSCHLÄGIGE DOKUMENTE | | | KLASSIFIKATION DER ANMELDUNG (Int.Cl.3) |
|---|---|---|---|

| Kategorie | Kennzeichnung des Dokuments mit Angabe, soweit erforderlich, der maßgeblichen Teile | betrifft Anspruch | |
|---|---|---|---|
| A,D | DE - A1 - 2 601 146 (KOPPERS)<br>* Anspruch 1 * | 1 | C 07 C 39/08<br>C 07 C 37/05 |
| A | US - A - 3 840 606 (VANLERBERGHE)<br>* Spalte 4, Zeilen 39 bis 45 * | 1 | |

**RECHERCHIERTE SACHGEBIETE (Int. Cl.3)**

C 07 C 37/05
C 07 C 39/08

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, Übereinstimmendes Dokument

| X | Der vorliegende Recherchenbericht wurde für alle Patentansprüche erstellt. |
|---|---|

| Recherchenort<br>Berlin | Abschlußdatum der Recherche<br>27-05-1980 | Prüfer<br>KNAACK |
|---|---|---|

EPA form 1503.1 06.78